(19) Europäisches Patentamt
European Patent Office
Office européen des brevets

(11) Publication number: **0 051 280 B1**

(12) # EUROPEAN PATENT SPECIFICATION

(45) Date of publication of patent specification: **11.07.84**

(21) Application number: **81109244.4**

(22) Date of filing: **29.10.81**

(51) Int. Cl.³: **C 07 H 15/24,** A 61 K 31/70, C 07 H 13/04 //C07H15/04

(54) **Anthracycline glycosides, process for the preparation thereof, intermediate compounds and their preparation and pharmaceutical compositions.**

(30) Priority: **01.11.80 GB 8035204**

(43) Date of publication of application: **12.05.82 Bulletin 82/19**

(45) Publication of the grant of the patent: **11.07.84 Bulletin 84/28**

(84) Designated Contracting States: **AT BE CH DE FR GB IT LI LU NL SE**

(56) References cited: **FR - A - 2 264 555**

(73) Proprietor: **FARMITALIA CARLO ERBA S.p.A.**
**Via Carlo Imbonati n.24**
**I-20159 Milan (IT)**

(72) Inventor: **Bargiotti, Alberto**
**Via Donati n. 8**
**Milan (IT)**
Inventor: **Cassinelli, Giuseppe**
**Via G. Matteotti n. 13**
**Milan (IT)**
Inventor: **Penco, Sergio**
**Via Crimea n. 13**
**Milan (IT)**
Inventor: **Arcamone, Federico**
**Via 4 Novembre 26**
**Nerviano (Milan) (IT)**
Inventor: **Casazza, Anna Maria**
**Via Guido Reni n. 26**
**Milan (IT)**

(74) Representative: **Hansen, Bernd, Dr.rer.nat. et al,**
**Hoffmann, Eitle & Partner Patentanwälte**
**Arabellastrasse 4**
**D-8000 München 81 (DE)**

**EP 0 051 280 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to anthracycline glycosides, to processes for their preparation, to pharmaceutical compositions containing them and to diamino-tetradeoxy sugar derivatives used in their preparation as well as a method to prepare those intermediate derivatives. The invention provides anthracycline glycosides of the general formula I and their pharmaceutically acceptable acid addition salts:

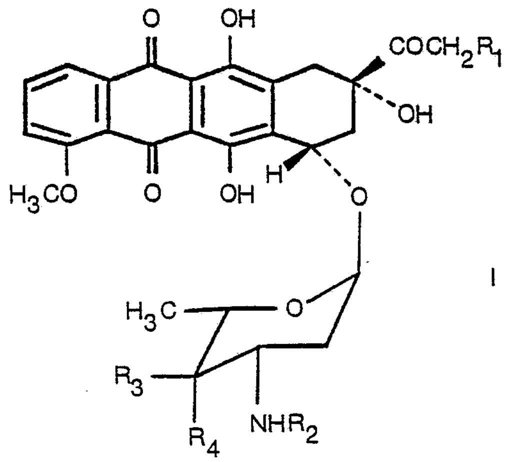

I

in which $R_1$ represents a hydrogen atom or a hydroxy group, $R_2$ represents a hydrogen atom or an acyl group, one of $R_3$ and $R_4$ represents an amino or an acylamino group and the other of $R_3$ and $R_4$ represents a hydrogen atom.

Amongst the anthracycline glycosides of the general formula I are:

4′-amino-4′-deoxy-daunorubicin (I—A) (I:$R_1$=$R_3$=H, $R_4$=$NH_2$),
4′-deoxy-4′-epi-trifluoroacetamido-daunorubicin (I—B) (I:$R_1$=$R_2$=$R_4$=H, $R_3$=$NHCOCF_3$),
4′-amino-4′-deoxy-4′-epi-daunorubicin (I—C) (I:$R_1$=$R_2$=$R_4$=H, $R_3$=$NH_2$),
4′-amino-4′-deoxy-doxorubicin (I—D) (I:$R_1$=OH, $R_2$=$R_3$=H, $R_4$=$NH_2$) and
4′-amino-4′-deoxy-4′-epi-doxorubicin (I—E) (I:$R_1$=OH, $R_2$=$R_4$=H, $R_3$=$NH_2$).

The anthracycline glycosides of the general formula I are useful therapeutic agents for treating certain mammalian tumours.

The invention further provides a process for the preparation of compounds of the general formula I, the process comprising condensing daunomycinone with 2,3,4,6-tetradeoxy-3,4-ditrifluoroacetamide-L-lyxo-hexopyranosyl chloride (III—A) or with 2,3,4,6-tetradeoxy-3,4-ditrifluoroacetamido-L-arabino-hexopyranosyl chloride (III—B) to give respectively the protected glycosides IV—A (I:$R_1$=$R_3$=H, $R_2$=$COCF_3$, $R_4$=NH—$COCF_3$) and IV—B (I: $R_1$=$R_4$=H, $R_2$=$COCF_3$, $R_3$=NH—$COCF_3$)

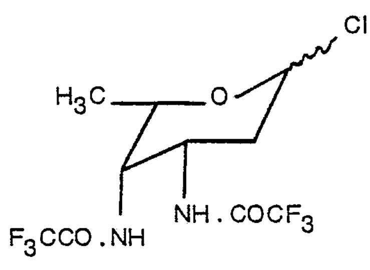

III—A

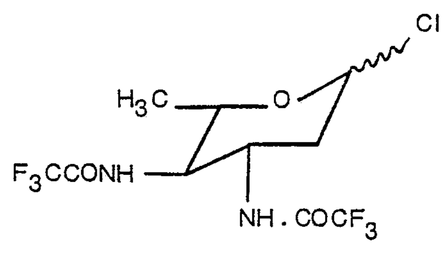

III—B

and selectively removing the protecting groups to give first the daunorubicin derivatives I in which $R_2$ represents a trifluoroacetyl group and then the daunorubicin derivatives I in which $R_2$ represents a hydrogen atom, and optionally 14-brominating the daunorubicin derivatives and hydrolysing the 14-bromo-daunorubicin derivatives to obtain the corresponding doxorubicin derivatives. The condensation is effected in the presence of a silver salt, preferably silver trifluoromethane sulphonate as catalyst, according to the procedure described in US—PS 4,112,076 of the applicant. The optional bromination and hydrolysis may be effected according to the procedure described in US—PS 4,122,076 of the applicant.

The invention also provides a pharmaceutical composition comprising a compound of the general

formula I or a pharmaceutically acceptable salt thereof in admixture with a pharmaceutically acceptable diluent or carrier.

The sugar derivatives III—A and III—B are also within the scope of the invention.

The starting material for the preparation of the intermediates III—A and III—B is methyl 2,3,6-trideoxy-3-trifluoroacetamido-α-L-threo-hexopyranosid-4-ulose (VI), a known compound described in US—PS 4,039,663 of the applicant.

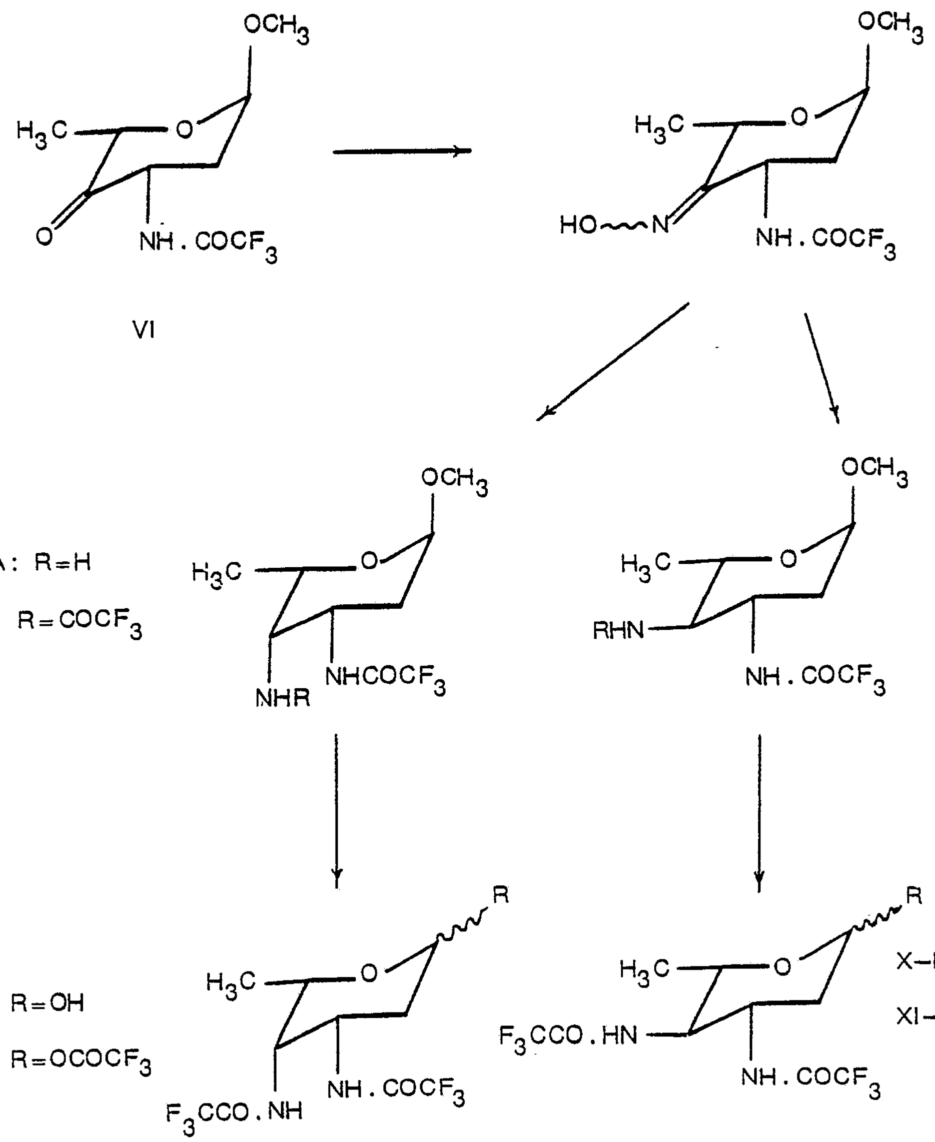

VI

VII

VIII—A: R=H

IX—A: R=COCF$_3$

VIII—B: R=H

IX—BR=COCF$_3$

X—A: R=OH

XI—A: R=OCOCF$_3$

X—B: R=OH

XI—B: R=OCOCF$_3$

Treatment of compound VI with hydroxylamine hydrochloride in pyridine gives the oxime VII, reduction of which in methanol in the presence of Raney-Nickel as catalyst gives a mixture of the lyxo-(VIII—A) and arabino-(VIII—B) sugar derivatives, which are separated after column chromatography. N-trifluoroacetylation followed by mild acid hydrolysis affords compound X—A and X—B respectively. Subsequent O-trifluoroacetylation and treatment with dry hydrogen chloride in anhydrous diethyl ether gives the 1-chloro-derivatives III—A and III—B respectively.

The invention is illustrated by the following Examples.

Example 1

Preparation of methyl 2,3,4,6-tetradeoxy-3,4-ditrifluoroacetamido-α-L-lyxo-hexopyranoside (IX—A) and methyl 2,3,4,6-tetradeoxy-3,4-ditrifluoroacetamido-α-L-arabino-hexopyranoside (IX—B)

Methyl 2,3,6-trideoxy-4-trifluoroacetamido-α-threohexopyranosid-4-ulose (V, 2.5 g, 1 mmol) was added to a solution of hydroxylamine hydrochloride (4.2 g, 60 mmol) in pyridine (20 ml) and methanol (20 ml). The reaction mixture was stirred at room temperature for 1 hour, then diluted with chloroform (160 ml) and water (100 ml). The organic phase was separated off, washed with water and concentrated under vacuum to give VIII, a mixture of syn- and anti-oximes, as a syrup. The syrup was dissolved in dry methanol (200 ml) and hydrogenated in the presence of Raney-Nickel at 10 atmospheres for 4 hours. The filtered solution was evaporated to dryness and the resulting syrup was chromatographed on a silica-gel column. Elution with 2% of methanol in chloroform gave a good separation of the intermediates VIII—A and VIII—B. These were N-trifluoroacetylated, in methylene dichloride with trifluoroacetic anhydride, to give respectively methyl 2,3,4,6-tetradeoxy-3,4-ditrifluoro-acetamido-$\alpha$-L-lyxo-hexopyranoside (IX—A, 1.2 g, 34%) as a white solid: m.p. 118—119°C, $[\alpha]_D^{20°}$ −94° (c=1, $CHCl_3$), and methyl 2,3,4,6-tetradeoxy-3,4-ditrifluoroacetamido-$\alpha$-L-arabinohexopyranoside (IX—B, 1,25 g, 36%) as a white solid: m.p. 231—232°C $[\alpha]_D^{20°}$ −220° (c=1, $CHCl_3$).

The structures of compound IX—A and IX—B were assigned on the basis of PMR studies in acetone-$d_6$ at 270 MHz. The PMR spectrum of IX—A showed absorptions at 1.13 (d, $CH_3$—C-5), 3.35 (s, $OCH_3$), 4.22 (qd, H—C-5), 4.42 (three d, H—C-4), 4.51 (four d, H—C-3) and 4.76 $\delta$ (dd, H—C-1), while that of IX—B showed absorptions at: 1.19 (d, $CH_3$—C-5), 3.32 (s, $OCH_3$), 3.76 (three d, H—C-4), 4.02 (dq, H—C-5), 4.47 (four d, H—C-3) and 4.81 $\delta$ (dd, H—C-1).

## Example 2

Preparation of 2,3,4,6-tetradeoxy-3,4-di-trifluoroacetamido-L-lyxo-hexopyranosyl chloride (III—A)

A solution compound IX—A (0.7 g, 2 mmol, prepared as described in Example 1), in acetic acid (20 ml) and water (45 ml), was heated for 3 hours at 100°C. The reaction mixture was evaporated to dryness under vacuum to give 2,3,4,6-tetradeoxy-3,4-ditrifluoroacetamido-L-lyxo-hexose (X—A) as a syrup. This was dissolved in methylene dichloride (15 ml) and treated at 0°C with trifluoroacetic anhydride (4 ml). After 2 hours at 0°C and 1 hour at room temperature, the reaction mixture was evaporated to give the corresponding 1-O-trifluoroacetate (XI—A) as a syrup, which was directly dissolved in anhydrous diethyl ether (20 ml). The solution was saturated at 0°C with dry hydrogen chloride. After standing at 0°C overnight, the reaction mixture was evaporated in vacuum to give the title compound suitable for the subsequent coupling reaction without further purification.

## Example 3

Preparation of 2,3,4,6-tetradeoxy-3,4-ditrifluoroacetamido-L-arabino-hexopyranosyl chloride (III—B)

Starting from methyl 2,3,4,6-tetradeoxy-3,4-ditrifluoroacetamido-L-arabino-hexopyranoside (IX—B, prepared as described in example 1) the title compound was prepared following the procedure described in Example 2.

## Example 4

Preparation of 4′-amino-4′-deoxy-daunorubicin (I—A) (XOO-0132)

Coupling of daunomycinone (0.9 g, 2.26 mmol) in dry methylene dichloride (105 ml) with 2,3,4,6-tetradeoxy-3,4-ditrifluoroacetamido-L-lyxo-hexopyranosyl chloride (III—A, 0.645 g, 1.8 mmol) prepared as described in Example 2, in the presence of Molecular Sieve (4 A—Merck, 6 g) was performed using silver trifluoromethane sulphonate (0.58 g in 15 ml of diethyl ether) as catalyst. After 1 hour under vigorous stirring at room temperature, the reaction mixture was treated with a saturated aqueous solution of sodium hdyrogen carbonate, and the organic phase was then separated off and evaporated under vacuum. Chromatographic purification of the crude residue on a silica-gel column, using a 95:5 by volume chloroform:acetone mixture as eluent, gave 4′-trifluoroacetamido-4′-deoxy-N-trifluoroacetyldaunorubicin (IV—A, 0.94 g 72%): m.p. 189—190°C (with decomposition: $[\alpha]_D^{20°}$ + 385° (c=0.05, MeOH). The PMR spectrum (acetone-$d_6$) showed absorptions at: 1.26 (d, $CH_3$—C-5′), 2.40 (s, $CH_3CO$), 4.00 (s, $CH_3$—O—C-4), 5.12 (broad s, H—C-7), 5.48 (dd, H—C-1′), 12.80 and 13.70 $\delta$ (two s, phenolic OHs).

A solution of IV—A (0.36 g, 0.05 mmol) in acetone (10 ml) and 0.2 N aqueous sodium hydroxide (30 ml) was stirred under nitrogen at room temperature. After two hours the reaction mixture was acidified (pH 3.5) with aqueous hydrochloric acid and then extracted with chloroform to eliminate some impurities. The aqueous phase, adjusted to pH 8, was extracted with chloroform and the extract was washed with water, dried over anhydrous sodium sulphate and concentrated to a small volume. Acidification (pH 4.5) with methanolic hydrogen chloride, followed by addition of diethyl ether, gave 4′-amino-4′-deoxy-daunorubicin (I—A, 0.21 g 74%) as its hydrochloride: m.p. 161—162°C (with decomposition), $[\alpha]_D^{23°}$ + 252° (c=0.05 in methanol).

## Example 5

Preparation of 4′-deoxy-4′-epi-trifluoroacetamido-daunorubicin (I—B) (XOO-0136)

Coupling of daunomycinone with 2,3,4,6-tetradeoxy-3,4-ditrifluoroacetamido-L-arabino-hexopyranosyl chloride (III—B, prepared as described in Example 3), following the procedure described in Example 4, gave 4′-deoxy-4′-epi-trifluoroacetamido-N-trifluoroacetyl-daunorubicin IV—B): m.p. 165—166° (with decomposition), $[\alpha]_D^{20°}$ + 145° (c=0.05, $CH_3OH$). The PMR spectrum (acetone-$d_6$) showed absorptions at 1.36 (d, $CH_3$—C-5′), 2.42 (s, $COCH_3$), 3.6—4.0 (m, H—C-4′), 4.07 (s, $CH_3$—O—C-4), 5.25 (broad s, H—C-7), 5.59 (broad s, H—C-1′), 12.90 and 13.77 $\delta$ (two s, phenolic OHs). Treatment of IV—B (0.28 g, 0.4 mmol) in acetone (8 ml) with 0.2 N aqueous sodium hydroxide (24 ml) under nitrogen for 30 minutes at room temperature, gave after the work-up described in Example 4, the title compound I—B (0.21 g, 85%) as its hydrochloride: m.p. 168—169°C (with decomposition), $[\alpha]_D^{20°}$+ 213° (c=0.05, in methanol).

Example 6

Preparation of 4′-amino-4′-deoxy-4′-epi-daunorubicin (I—C)

Removal of the trifluoroacetyl protecting group from Compound I—B (prepared as described in Example 5), following the procedure described in Example 4, gave the title compound, isolated as its hydrochloride.

Example 7

Preparation of 4′-amino-4′-deoxy-doxorubicin (I—D)

Chemical transformation of I—A (prepared as described in Example 4) into the title compound I—D was performed by 14-bromination followed by hydrolysis, according to the procedure described in the US—PS 4,122076

*Biological activity*

On HeLa cells cloning efficiency in vitro, the two new derivatives were less cytotoxic than daunorubicin (Table 1). Both compounds resulted more active than daunorubicin in a preliminary test against P 388 leukemia in mice (Table 2). In particular, XOO-0132 was less toxic than daunorubicin and highly active at the maximal dose tested of 13.5 mg/kg.

TABLE 1

Activity on HeLa cells cloning efficiency in vitro.

Treatment for 24 hours.

| Compound | Dose (ng/ml) | % of controls | $ID_{50}$ (ng/ml) |
|---|---|---|---|
| Daunorubicin[a] | 12.5 | 49—48 | 12—12 |
| | 6.2 | 73—87 | |
| | 3.1 | 88—98 | |
| XOO-0132 (I—A) | 100 | 0 | 35 |
| | 25 | 70 | |
| | 6.2 | 98 | |
| XOO-0136 (I—B) | 1600 | 0 | 180 |
| | 400 | 21 | |
| | 100 | 211 | |

[a]Data of 2 experiments.

TABLE 2

Activity against P 388 ascitic leukemia. Treatment i.p. on day 1 after tumour inoculation

| Compound | Dose (mg/kg) | T/C (%) | Toxicity deaths |
|---|---|---|---|
| Daunorubicin | 2.9 | 181 | 0/10 |
| | 4.4 | 140 | 9/10 |
| | 6.6 | 118 | 10/10 |
| XOO-0132 (I—A) | 6 | 181 | 0/10 |
| | 9 | 190 | 0/10 |
| | 13.5 | 222 | 0/10 |
| XOO-0136 (I—B) | 10 | 145 | 0/10 |
| | 20 | 154 | 0/10 |
| | 40 | 163 | 2/10 |

**Claims for the Contracting States; BE CH DE FR GB IT LI LU NL SE**

1. Anthracycline glycosides of the general formula I:

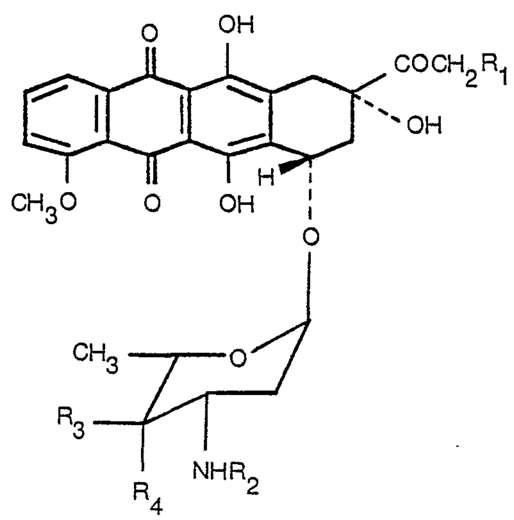

(I)

wherein $R_1$ represents a hydrogen atom or a hydroxy group, $R_2$ represents a hydrogen atom or an acyl group; one of $R_3$ and $R_4$ represents an amino or an acylamino group and the other of $R_3$ or $R_4$ represents a hydrogen atom, and their pharmaceutically acceptable acid addition salts.

2. A compound according to claim 1, which is 4′-amino-4′-deoxy-daunorubicin hydrochloride.

3. A compound according to claim 1, which is 4′-deoxy-4′-epi-trifluoracetamido-daunorubicin hydrochloride.

4. A compound according to claim 1 which is 4′-amino-4′-deoxy-4′-epi′-daunorubicin hydrochloride.

5. A compound according to claim 1, which is 4′-amino-4′-deoxy-doxorubicin hydrochloride.

6. A compound according to claim 1, which is 4′-amino-4′-deoxy-4′-epi-doxorubicin hydrochloride.

7. A process for the preparation of anthracycline glycosides of the general formula I according to claim 1, wherein $R_1$ represents a hydrogen atom, characterized by

(a) condensing daunomycinone with 2,3,4,6-tetradeoxy-3,4-ditrifluoroacetamido-L-lyxo-hexopyranosyl chloride or with 2,3,4,6-tetra-deoxy-3,4-ditrifluoroacetamido-L-arabino-hexopyranosyl chloride, to yield respectively protected glycosides; and

(b) selectively removing the protecting groups to give first the daunorubicin derivatives I; in which

$R_2$ represents a trifluoroacetyl group, and then the daunorubicin derivatives I in which $R_2$ represents a hydrogen atom.

8. A process for the preparation of anthracycline glycosides of the general formula I according to claim 1, wherein $R_1$ represents a hydroxyl group, characterized by 14-brominating the daunorubicin derivatives as obtained according to claim 7 and hydrolyzing the 14-bromo-daunorubicin derivatives to obtain the corresponding doxorubicin derivatives.

9. A process according to claims 7 and 8, characterized in that the condensation is effected in the presence of a silver salt.

10. A process according to claim 9, characterized in that the condensation is effected in the presence of silver-trifluoromethane sulphonate as catalyst.

11. Intermediate compound for the preparation of anthracycline glycosides according to claim 1, characterized in that it represents 2,3,4,6-tetra-deoxy-3,4-ditrifluoroacetamido-L-lyxo-hexopyranosyl chloride.

12. Intermediate compound for the preparation of anthracycline glycosides according to claim 1, characterized in that it represents 2,3,4,6-tetra-deoxy-3,4-ditrifluoroacetamido-L-arabino-hexopyranosyl chloride.

13. A process for the preparation of the intermediate compounds according to claims 11 and 12, characterized in that

(a) 2,3,6-trideoxy-3-trifluoroacetamido-α-L-threohexopyranosid-4-ulose is treated with hydroxyl amine hydrochloride in pyridine to give the respective oxime, which is reduced in methanol in the presence of Raney-Nickel as catalyst to yield a mixture of lyxo- and arabino-sugar derivatives;

(b) the obtained sugar derivatives are separated by column chromatography;

(c) the compounds obtained are N-trifluoroacetylated, and after mild acid hydrolysis the respective hydroxy derivatives are obtained;

(d) subsequently O-trifluoro-acetylation is carried out, followed by treatment with dry hydrogen chloride in anhydrous diethyl ether to yield the respective 1-chloroderivatives.

14. A pharmaceutical composition comprising a therapeutically effective amount of a compound according to claim 1, in combination with a carrier thereof.

**Claims for the Contracting State: AT**

1. A process for the preparation of anthracycline glycosides of the general formula I

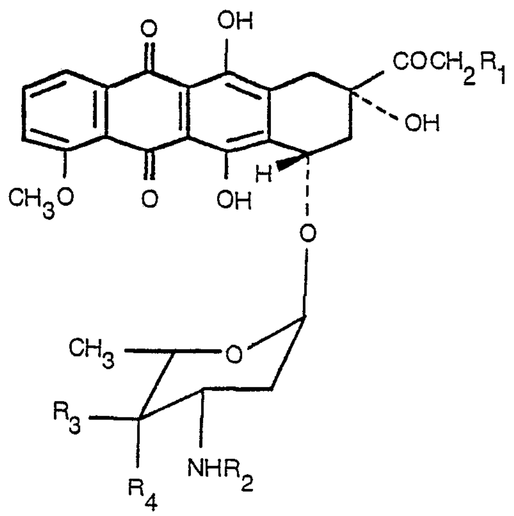

(I)

wherein $R_1$ represents a hydrogen atom, $R_2$ represents a hydrogen atom or an acyl group; one of $R_3$ and $R_4$ represents an amino or an acylamino group and the other of $R_3$ or $R_4$ represents a hydrogen atom, and their pharmaceutically acceptable acid addition salts, characterized by

(a) condensing daunomycinone with 2,3,4,6-tetradeoxy-3,4-ditrifluoroacetâmido-L-lyxo-hexopyranosyl chloride or with 2,3,4,6-tetra-deoxy-3,4-ditrifluoroacetamido-L-arabino-hexapyranosyl chloride, to yield respectively protected glycosides; and

(b) selectively removing the protecting groups to give first the daunorubicin derivatives I, in which $R_2$ represents a trifluoroacetyl group, and then the daunorubicin derivatives I in which $R_2$ represents a hydrogen atom, and

for compounds wherein $R_1$ represents a hydroxyl group and the residues $R_2$, $R_3$ and $R_4$ are the same as above,

14-brominating of daunorubicin derivatives as obtained according to step (b), and

hydrolyzing the 14-bromo-daunorubicin derivatives to obtain the corresponding doxorubicin derivatives.

2. A process for preparing the intermediates 2,3,4,6-tetradeoxy-3,4-ditrifluoroacetamido-L-lyxo-hexopyranosyl chloride and 2,3,4,6-tetra-deoxy-3,4-ditrifluoroacetamido-L-arbino-hexopyranosyl chloride for the preparation of anthracycline glycosides according to claim 1, characterized in that

(a) 2,3,6-trideoxy-3-trifluoroacetamido-$\alpha$-L-threo-hexopyranosid-4-ulose is treated with hydroxyl amine hydrochloride in pyridine to give the respective oxime, which is reduced in methanol in the presence of Raney-Nickel as catalyst to yield a mixture of lyxo- and arabino-sugar derivatives;

(b) the obtained surgar derivatives are separated by column chromatography;

(c) the compounds obtained are N-trifluoroacetylated, and after mild acid hydrolysis the respective hydroxy derivatives are obtained;

(d) subsequently O-trifluoro-acetylation is carried out, followed by treatment with dry hydrogen chloride in anhydrous diethyl ether to yield the respective 1-chloro derivatives.

3. A process according to claim 1, characterized in that the condensation is effected in the presence of a silver salt.

4. A process according to claim 1, characterized in that the condensation is effected in the presence of silver-trifluoromethane sulphonate as catalyst.

**Revendications pour les états Contractants: BE CH DE FR GB IT LI NL LU SE**

1. Dérivés glycosidiques d'anthracycline de formule générale I:

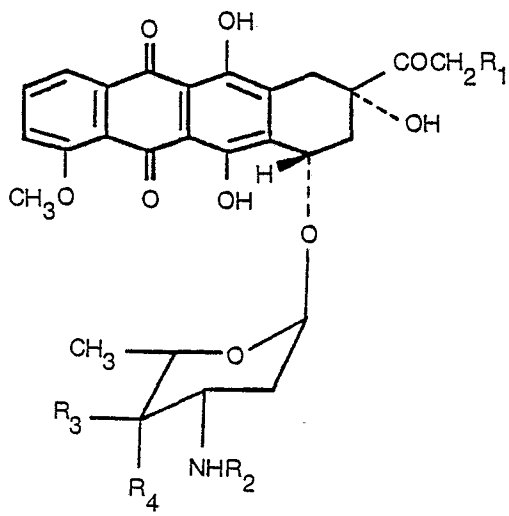

(I)

dans laquelle $R_1$ représente un atome d'hydrogène ou un groupe hydroxy, $R_2$ représente un atome d'hydrogène ou un groupe acyle; l'un de $R_3$ et $R_4$ représente un groupe amino ou acylamino et l'autre de $R_3$ ou $R_4$ représente un atome d'hydrogène, et leurs sels d'addition acide pharmaceutiquement acceptables.

2. Composé selon la revendication 1 qui est le chlorhydrate de 4'-amino-4'-désoxy-daunorubicine.

3. Composé selon la revendication 1 qui est le chlorhydrate de 4'-désoxy-4'-épi-trifluoro-acétamido-daunorubicine.

4. Composé selon la revendication 1 qui est le chlorhydrate de 4'-amino-4'-désoxy-4'-épi-daunorubicine.

5. Composé selon la revendication 1 qui est le chlorhydrate de 4'-amino-4'-désoxy-doxorubicine.

6. Composé selon la revendication 1 qui est le chlorhydrate de 4'-amino-4'-désoxy-4'-épi-doxorubicine.

7. Procédé pour la préparation des dérivés glycosidiques d'anthracycline de formule générale I selon la revendication 1 dans laquelle $R_1$ représente un atome d'hydrogène, caractérisé en ce que:

(a) on condense la daunomycine avec le chlorure de 2,3,4,6-tétradésoxy-3,4-ditrifluoro-acétamido-L-lyxo-hexopyranosyle ou avec le chlorure de 2,3,4,6-tétradésoxy-3,4-ditrifluoro-acétamido-L-arabino-hexopyranoxyle pour obtenir respectivement des glycosides protégés;

(b) on élimine sélectivement les groupes protecteurs pour obtenir les dérivés I de daunorubicine dans lesquels $R_2$ représente un group trifluoroacétyle et ensuite les dérivés I de daunorubicine dans lequels $R_2$ représente un atome d'hydrogène.

8. Procédé pour la préparation des dérivés glycosidiques d'anthracycline de formule générale I selon la revendication 1, dans laquelle $R_1$ représente un groupe hydroxyle, caractérisé en ce qu'on soumet à une bromation en 14 les dérivés de daunorubicine obtenus selon la revendication 7 et on hydrolyse les dérivés de 14-bromo-daunorubicine pour obtenir les dérivés de doxorubicine correspondants.

9. Procédé selon l'une quelconque des revendications 7 et 8 caractérisé en ce qu'on effectue la condensation en présence d'un sel d'argent.

10. Procédé selon la revendication 9 caractérisé en ce qu'on effectue la condensation en présence de trifluorométhane sulfonate d'argent comme catalyseur.

11. Composé intermédiaire pour la préparation des dérivés glycosidiques d'anthracycline selon la revendication 1 caractérisé en ce qu'il est constitué par le chlorure de 2,3,4,6-tétradésoxy-3,4-ditrifluoroacétamido-L-lyxo-hexopyranosyle.

12. Composé intermédiaire pour la préparation de dérivés glycosidiques d'anthracycline selon la revendication 1 caractérisé en ce qu'il est constitué par le chlorure de 2,3,4,6-tétradésoxy-3,4-ditrifluoroacétamido-L-arabino-hexopyranosyle.

13. Procédé pour la préparation des composés intermédiaires selon les revendications 11 et 12 caractérisé en ce que:

(a) on traite du 2,3,6-tridésoxy-3-trifluoroacétamido-$\alpha$-L-thréo-hexopyranoside-4-ulose avec du chlorhydrate d'hydroxylamine dans de la pyridine pour obtenir l'oxime correspondant que l'on réduit dans du méthanol en présence de nickel de Raney comme catalyseur pour obtenir un mélange de dérivés lyxo- et arabino-sucre;

b) on sépare les dérivés sucre obtenus par chromatographie sur colonne;

c) on soumet à une opération de N-trifluoroacétylation les composés obtenus et aprés une hydrolyse acide douce on obtient les dérivés hydroxy respectifs;

d) on effectue ensuite une O-trifluoro-acétylation, suivie d'un traitement à l'aide d'acide chlorhydrique sec dans de l'éther diéthylique anhydre, pour obtenir les 1-chloro-dérivés respectifs.

14. Composition pharmaceutique caractérisée en ce qu'elle comprend une quantité efficace thérapeutiquement d'un composé selon la revendication 1 en combinaison avec un porteur de ce dernier.

**Revendications pour l'état contractant: AT**

1. Procédé pour la préparation de dérivés glycosidiques d'antrhacycline de formule générale I

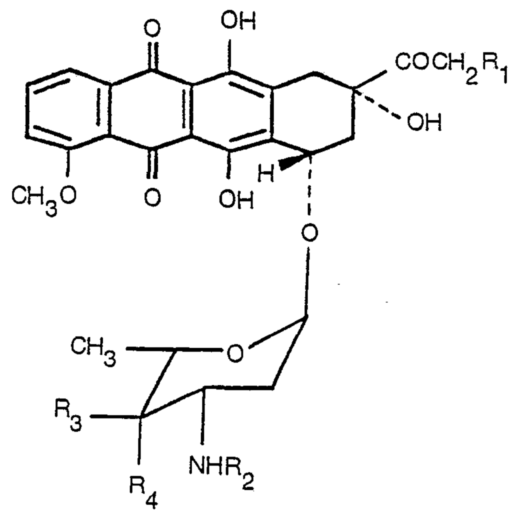

(I)

dans laquelle $R_1$ représente un atome d'hydrogène $R_2$ représente un atome d'hydrogène ou un groupe acyle, l'un de $R_3$ et $R_4$ représente un groupe amino ou acylamino et l'autre de $R_3$ ou $R_4$ représente un atome d'hydrogène, et leurs sels d'addition acide pharmaceutiquement acceptables, caractérisé en ce que:

(a) on condense la daunomycinone avec le chlorure de 2,3,4,6-tétra-désoxy-3,4-ditrifluoroacétamido-L-lyxo-hexopyranosyle ou avec le chlorure de 2,3,4,6-tétra-désoxy-3,4-ditrifluoroacétamido-L-arabino-hexopyranosyle pour obtenir respectivement des glycosides protégés;

(b) on élimine sélectivement les groupes protecteurs pour obtenir d'abord les dérivés I de daunorubicine dans lesquels $R_2$ représente un groupe trifluoroacétyle et ensuite les dérivés I de daunorubicine dans lesquels $R_2$ représente un atome d'hydrogène, et

pour les composés dans lesquels $R_1$ représente un groupe hydroxyle et les résidus $R_2$, $R_3$ et $R_4$ sont les mêmes que ci-dessus,

on soumet à une bromation en 14 les dérivés de daunorubicine obtenus selon l'opération (b), et

on hydrolyse les dérivés de 14-bromo-daunorubicine pour obtenir les dérivés de doxorubicine correspondants.

2. Procédé pour préparer les produits intermédiaires chlorure de 2,3,4,6-tétradésoxy-3,4-ditrifluoroacétamido-L-lyxo-hexopyranosyle et chlorure de 2,3,4,6-tétra-désoxy-3,4-ditrifluoroacétamido-L-arabino-hexopyranosyle en vue de la préparation des dérivés glycosidiques d'anthracycline selon la revendication 1, caractérisé en ce que:

(a) on traite du 2,3,6-tridésoxy-3-trifluoroacétamido-$\alpha$-L-thréo-hexopyranoside-4-ulose avec du chlorhydrate d'hydroxylamine dans de la pyridine pour obtenir l'oxime correspondant que l'on réduit dans du méthanol en présence de nickel de Raney comme catalyseur pour obtenir un mélange de dérivés lyxo- et arabino-sucre;

(b) on sépare les dérivés sucre obtenus par chromatographie sur colonne;

(c) on soumet à une opération de N-trifluoroacétylation les composés obtenus et après une hydrolyse acide douce on obtient les dérivés hydroxy respectifs;

(d) on effectue ensuite une O-trifluoro-acétylation, suivie d'un traitement à l'aide d'acide chlorhydrique sec dans de l'éther diéthylique anhydre pour obtenir les 1-chloro-dérivés respectifs.

3. Procédé selon la revendication 1 caractérisé en ce qu'on effectue la condensation en présence d'un sel d'argent.

4. Procédé selon la revendication 1 caractérisé en ce qu'on effectue la condensation en présence de trifluorométhane sulfonate d'argent comme catalyseur.

**Patentansprüche für die Vertragsstaaten: BE CH DE FR GB IT LI LU NL SE**

1. Anthracyclin-glyxcoside der allgemeinen Formel (I):

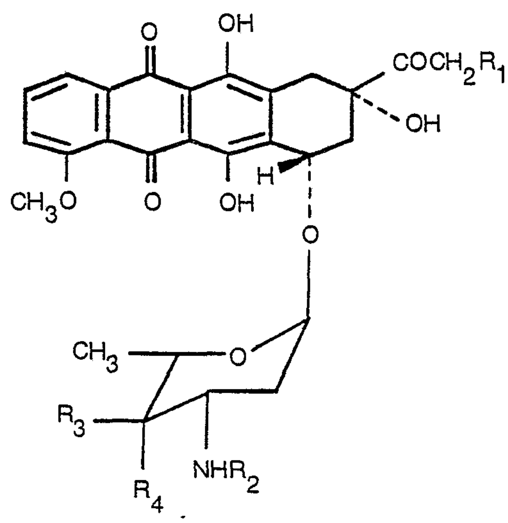

(I)

worin $R_1$ ein Wasserstoffatom oder eine Hydroxygruppe bedeutet, $R_2$ ein Wasserstoffatom oder eine Acylgruppe darstellt; einer der Substituenten $R_3$ und $R_4$ eine Amino- oder eine Acylaminogruppe bedeutet und der andere Substituent $R_3$ oder $R_4$ ein Wasserstoffatom darstellt, und deren pharmazeutisch annehmbare Säureadditionssalze.

2. 4'-Amino-4'-desoxy-daunorubicin-hydrochlorid.

3. 4'-Desoxy-4'-epi-trifluoroacetamido-daunorubicinhydrochlorid.

4. 4'-Amino-4'-desoxy-4'-epi-daunorubicin-hydrochlorid.

5. 4'-Amino-4'-desoxy-doxorubicin-hydrochlorid.

6. 4'-Amino-4'-desoxy-4'-epi-doxorubicin-hydrochlorid.

7. Verfahren zur Herstellung von Anthracyclin-glycosiden der allgemeinen Formel (I) gemäss Anspruch 1, worin $R_1$ ein Wasserstoffatom darstellt, gekennzeichnet durch

(a) Kondensieren von Daunomycinon mit 2,3,4,6-Tetradesoxy-3,4-ditrifluoroacetamido-L-lyxo-hexopyranosylchlorid oder mit 2,3,4,6-Tetradesoxy-3,4-ditrifluoroacetamido-L-arabino-hexopyranosylchlorid unter Erhalt der jeweiligen geschützten Glycoside; und

(b) selektive Entfernung der Schutzgruppen, wobei zunächst Daunorubicin-Derivate I, in welchen $R_2$ eine Trifluoroacetylgruppe darstellt, und dann die Daunorubicin-Derivate I, in welchen $R_2$ ein Wasserstoffatom bedeutet, erhalten werden.

8. Verfahren zur Herstllung von Anthracyclin-glycosiden der allgemeinen Formel (I) gemäss Anspruch 1, worin $R_1$ eine Hydroxylgruppe bedeutet gekennzeichnet durch 14-Bromierung der Daunorubicin-Derivate, wie sie in Anspruch 7 erhalten werden und Hydrolysieren der 14-Bromo-daunorubicin-Derivate unter Erhalt der entsprechenden Doxorubicin-Derivate.

9. Verfahren gemäss Ansprüchen 7 und 8, dadurch gekennzeichnet, dass die Kondensation in Gegenwart eines Silbersalzes ausgeführt wird.

10. Verfahren gemäss Anspruch 9, dadurch gekennzeichnet, dass die Kondensation in Gegenwart eines Silber-trifluoromethan-sulfonats als Katalysator erfolgt.

11. Zwischenverbindung zu Herstellung von Anthracyclinglycosiden gemäss Anspruch 1, dadurch

kennzeichnet, dass diese 2,3,4,6-Tetra-desoxy-3,4-ditrifluoroacetamido-L-lyxo-hexopyranosyl-chlorid darstellt.

12. Zwischenverbindung zur Herstellung von Anthracyclin-glycosiden gëmass Anspruch 1, dadurch gekennzeichnet, dass diese 2,3,4,6-Tetra-desoxy-3,4-ditrifluoroacetamido-L-arabino-hexopyranosylchlorid darstellt.

13. Verfahren zur Herstellung der Zwischenverbindungen gemäss Ansprüchen 11 und 12, dadurch gekennzeichnet, dass

(a) 2,3,6-Tridesoxy-3-trifluoroacetamido-alpha-L-threohexopyranosid-4-ulose mit Hydroxylaminhydrochlorid in Pyridin behandelt wird, wobei das entsprechende Oxim erhalten wird, welches in Methanol in Gegenwart von Raney-Nickel als Katalysator reduziert wird, wobei ein Gemisch der Lyxo- und Arabinozucker-Derivaten erhalten wird;

(b) die erhaltenen Zuckerderivate durch Säulenchromatografie aufgetrennt werden;

(c) die erhalten Verbindungen N-trifluoroacetyliert werden, wobei nach milder Säurehydrolyse die jeweiligen Hydroxyderivate erhalten werden;

(d) anschliessend eine O-Trifluoro-acetylierung, gefolgt von einer Behandlung mit trockenem Chlorwasserstoff in wasserfreiem Diethylether durchgeführt wird, wobei die jeweiligen 1-Chloroderivate erhalten werden.

14. Pharmazeutische Zubereitung, dadurch gekennzeichnet dass sie eine therapeutisch wirksame Menge einer Verbindung gemäss Anspruch 1 in Kombination mit einem Träger derselben umfasst.

**Patentansprüche für den Vertragsstaat: AT**

1. Verfahren zur Herstellung von Anthracyclinglycosiden der allgemeinen Formel (I)

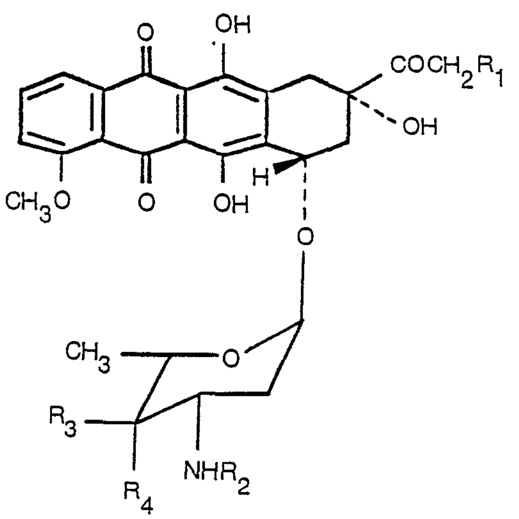

(I)

worin $R_1$ ein Wasserstoffatom darstellt, $R_2$ ein Wasserstoffatom oder eine Acylgruppe bedeutet, einer der Substituenten $R_3$ und $R_4$ eine Amino- oder Acylaminogruppe bedeutet, und der andere Substituent von $R_3$ oder $R_4$ ein Wasserstoffatom darstellt, und deren pharmazeutisch annehmbare Säureadditionssalze, gekennzeichnet durch

(a) Kondensieren von Daunomycinon mit 2,3,4,6-Tetra desoxy-3,4-ditrifluouracetamido-L-lyxo-hexopyranosylchlorid oder mit 2,3,4,6-Tetradesoxy-3,4-ditrifluoroacetamido-L-arabino-hexopyranosyl-chlorid unter Erhalt der jeweiligen geschützten Glycoside; und

(b) selektive Entfernung der Schutzgruppen, wobei zunächst Daunorubicin-Derivative I, in welchen $R_2$ eine Trifluoroactylgruppe darstellt, und dann die Daunorubicin-Derivative I, in welchen $R_2$ ein Wasserstoffatom bedeutet, erhalten werden, und für Verbindungen, worin $R_1$ eine Hydroxylgruppe darstellt und die Reste $R_2$, $R_3$ und $R_4$ die vorstehend angegebene Bedeutung haben,

14-Bromierung der Daunorubicin-Derivate, wie sie im Schritt (b) erhalten werden, und

Hydrolsieren der 14-Bromo-daunorubicin-Derivate unter Erhalt der entsprechenden Doxorubicin-Derivate.

2. Verfahren zur Herstellung der Zwischenverbindungen 2,3,4,6-Tetra-desoxy-3,4-ditrifluoroacetamido-L-lyxo-hexopyranosyl-chlorid und 2,3,4,6-Tetra-desoxy-3,4-ditrifluoroacetamido-L-arabino-hexopyranosyl-chlorid zur Herstellung der Anthracyclin-glycosiden gemäss Anspruch 1, dadurch gekennzeichnet, dass

(a) 2,3,6-Tridesoxy-3-trifluoroacetamido-alpha-L-threo-hexopyranosid-4-ulose mit Hydroxylaminhydrochlorid in Pyridin behandelt wird, wobei das entsprechende Oxim erhalten wird, welches in

Methanol in Gegenwart von Raney-Nickel als Katalysator reduziert wird, wobei ein Gemisch der Lyxo- und Arabinozucker-Derivate erhalten wird;

(b) die erhaltenen Zuckerderivate durch Säulenchromatografie aufgetrennt werden;

(c) die erhaltenen Verbindungen N-trifluoroacetyliert werden, wobei nach milder Säurehydrolyse die jeweiligen Hydroxyderivate erhalten werden;

(d) anschliessend eine O-Trifluoro-acetylierung, gefolgt von einer Behandlung mit trockenem Chlorwasserstoff in wasserfreiem Diethylether durchgeführt wird, wobei die jeweiligen 1-Chloroderivate erhalten werden.

3. Verfahren gemäss Anspruch 1, dadurch gekennzeichnet, dass die Kondensation in Gegenwart eines Silbersalzes erfolgt.

4. Verfahren gemäss Anspruch 1, dadurch gekennzeichnet, dass die Kondensation in Gegenwart von Silber-trifluoromethan-sulfonat als Katalysator erfolgt.